# EUROPEAN PATENT SPECIFICATION

(11) **EP 3 185 705 B1**
(45) Date of publication and mention of the grant of the patent: **23.10.2019**
(21) Application number: 15757213.2
(22) Date of filing: 26.08.2015
(51) Int. Cl.: A24F 47/00

(54) **METHOD FOR APPLYING HEAT CONDUCTING PATCHES TO A MATERIAL WEB**
VERFAHREN ZUR ANBRINGUNG WÄRMELEITENDER PFLASTER AUF EINE MATERIALBAHN
PROCÉDÉ D'APPLICATION DE TIMBRES CONDUCTEURS DE CHALEUR SUR UNE BANDE DE MATIÈRE

(30) Priority: 27.08.2014 EP 14182540
(43) Date of publication of application: 05.07.2017
(73) Proprietor: Philip Morris Products S.A., 2000 Neuchatel (CH)
(72) Inventor: GINDRAT, Pierre-Yves, 1907 Saxon (CH)
(74) Representative: Lupini, Stephen Antony
(86) International application number: PCT/EP2015/069568
(87) International publication number: WO 2016/030433

(56) References cited:
- WO-A2-2009/022232
- US-A1- 2007 215 168

## Description

The present invention relates to methods for applying heat conducting patches to a material web. In particular, the present invention relates to methods for applying heat conducting patches onto a web of smoking article wrapper material. The present invention also relates to methods for manufacturing multi-segment components for smoking articles and to smoking articles having one or more heat conducting elements.

A number of smoking articles in which tobacco is heated rather than combusted have been proposed in the art. An aim of such 'heated' smoking articles is to reduce known harmful smoke constituents of the type produced by the combustion and pyrolytic degradation of tobacco in conventional cigarettes. In one known type of heated smoking article, an aerosol is generated by the transfer of heat from a combustible heat source to a physically separate aerosol-forming substrate, such as tobacco. The aerosol-forming substrate may be located within, around or downstream of the combustible heat source. During smoking, volatile compounds are released from the aerosol-forming substrate by heat transfer from the combustible heat source and entrained in air drawn through the smoking article. As the released compounds cool, they condense to form an aerosol that is inhaled by the user. Typically, air is drawn into such known heated smoking articles through one or more airflow channels provided through the combustible heat source and heat transfer from the combustible heat source to the aerosol-forming substrate occurs by convention and conduction.

For example, WO-A-2009/022232 discloses a smoking article comprising a combustible heat source, an aerosol-forming substrate downstream of the combustible heat source, and a heat-conducting element around and in contact with a rear portion of the combustible heat source and an adjacent front portion of the aerosol-forming substrate.

The heat-conducting element in the smoking article of WO-A-2009/022232 transfers the heat generated during combustion of the heat source to the aerosol-forming substrate via conduction. The heat drain exerted by the conductive heat transfer significantly lowers the temperature of the rear portion of the combustible heat source so that the temperature of the rear portion is retained significantly below its self-ignition temperature.

To manufacture smoking articles with a heat-conducting element, it is known to cut and glue patches of heat conductive material onto a web of wrapper material at spaced intervals before wrapping the web around the smoking article so that one of the patches of heat conductive material forms a heat conducting element around the smoking article along part of its length.

For example, WO-A-2009/112257 discloses a process for applying heat conducting patches to a paper web whereby glue is applied to a web of aluminium foil, the web is cut into individual heat conducting patches on a cutting drum and the patches are transferred to the web of paper using a transfer drum. The spacing between adjacent patches on the paper web can be varied by changing the speed of the web of aluminium foil using pinch drive rollers and using a dancing roller to maintain tension in the web as its speed is varied.

However, when heat conducting patches are cut from a web of foil and applied at high speed, it can be difficult to accurately position the patches on the paper web, not least since the correct positioning of the patches requires precise control of the relative speeds of the two webs. In addition, individual patches of heat conducting material can fall from the cutting drum or the transfer drum prior to their application onto the web of paper, causing machine stoppages.

It would be desirable to provide an improved smoking article and an improved method for applying heat conducting patches onto a web of smoking article wrapper material.

According to a first aspect of the present invention, there is provided a smoking article comprising: a combustible heat source; an aerosol-forming substrate; a wrapper circumscribing at least the combustible heat source and the aerosol-forming substrate; and one or more heat conducting elements around a rear portion of the combustible heat source and an adjacent front portion of the aerosol-forming substrate, wherein at least one heat conducting element comprises a patch of thermally conductive printable medium printed onto the wrapper.

As used herein, the terms "upstream", "front", "downstream" and "rear" are used to describe the relative positions of components or portions of components of smoking articles of the invention in relation to the direction in which a user draws on the smoking articles during use.

Smoking articles of the invention comprise a mouth end and an opposed distal end. In use, a user draws on the mouth end of the smoking article. The mouth end is downstream of the distal end. The heat source is located at or proximate to the distal end.

As used herein, the term "length" is used to describe the dimension in the longitudinal direction of the smoking article.

As used herein, the term "patch" is used to describe a discrete area of material applied onto the web of smoking article material. This includes patches composed of a number of discrete or connected portions, for example arranged in a pattern, as well as patches composed of a single portion of additional material.

The smoking article of the present invention includes one or more heat conducting elements around a rear portion of the heat source and an adjacent front portion of the aerosol-forming substrate, wherein at least one of the one or more heat conducting elements comprises a patch of thermally conductive printable medium printed onto the wrapper.

Advantageously, smoking articles according to the invention require less machinery to manufacture and are easier to produce at high speed. Further, since the printed patch is formed directly onto the wrapper, there is no risk of it being misplaced during the manufacturing process. Furthermore, the appearance of smoking articles according to the present invention can be improved over known smoking articles.

The thermally conductive printable medium may comprise any suitable printable medium, such as ink or adhesive, or a combination thereof. Suitable inks may include, but are not limited to inks comprising a liquid vehicle (for example, water or an organic solvent) with a dispersed or dissolved heat conducting component. The heat conducting component may comprise any suitable heat conducting component, or combination of components. In certain embodiments, the heat conducting component comprises a thermally conductive carbon component, such as graphite. In preferred embodiments, the thermally conductive printable medium is a metal based ink. In such embodiments, the ink may comprise one or more of metal flakes, metal nanoparticles, metal complexes, metal salts, and metallo-organic compounds. Suitable metals include, but are not limited to steel, silver, copper, gold and aluminium. The thermally conductive printable medium may comprise a metal based ink formed from a mixture of two or more metals. For example, the thermally conductive printable medium may comprise an aluminium and zinc ink in the ratio of about 85:15 aluminium to zinc. In a preferred embodiment, the thermally conductive printable medium comprises an aluminium based ink..

The patch of thermally conductive printable medium may be of any suitable pattern. For example, each patch may comprise a plurality of linear or non-linear continuous strips or bands aligned substantially in the longitudinal direction. The area of printed material forming the patch may be less than the total area of the patch. The area of printed material forming each patch is at least 50% of the total area of the patch. In certain embodiments, the area of printed material forming each patch is at least 80% of the total area of the patch. More preferably, each printed patch comprises a single block of printed material. That is, each printed patch is formed by printing thermally conductive printable medium over substantially the entire area of the patch.

The patch of thermally conductive printable medium may be of any suitable size or shape. For example, the printed patch may have a length of from about 2 mm to about 35 mm, preferably from about 3 mm to about 18 mm, more preferably from about 5 mm to about 13 mm, most preferably for about 5 mm to about 11 mm.

Smoking articles according to the invention may comprise one or more heat-conducting elements around a rear portion of the heat source and an adjacent front portion of the aerosol-forming substrate. The one or more heat-conducting elements may extend partly around the circumferences of the heat source or the aerosol-forming substrate, or both. Alternatively, the one or more heat-conducting elements may extend around the entire circumference of the heat source or the aerosol-forming substrate, or both. In a preferred embodiment, the patch comprises a single block of thermally conductive printed medium circumscribing the smoking article. Preferably, the one or more heat-conducting element forms a continuous sleeve that circumscribes the smoking article along part of its length.

The one or more heat-conducting elements may comprise a single heat-conducting element printed onto an inner surface of the wrapper. In such embodiments, the heat-conducting element may be in direct contact with the rear portion of the heat source and with the front portion of the aerosol-forming substrate. Alternatively, smoking articles according to the invention may comprise a single heat-conducting element printed onto an outer surface of the wrapper.

The one or more heat-conducting elements may comprise a first heat-conducting element around and in direct contact with the rear portion of the heat source and an adjacent front portion of the aerosol-forming substrate and a second heat-conducting element around at least a portion of the first heat-conducting element. The second heat-conducting element is radially separated from the first heat-conducting element by the wrapper. That is, the second heat-conducting element is spaced apart from the underlying first heat-conducting element in a radial direction by the wrapper, such that there is no direct contact between that part of the second heat-conducting element and the first heat-conducting element. In such embodiments, the wrapper is wrapped around the smoking article over the first heat-conducting element, and the second heat-conducting element is applied on top of at least a portion of the wrapper. The second heat-conducting element may therefore be provided at the outside of the smoking article, such that the second heat-conducting element is visible on the external surface of the smoking article. Alternatively, an additional wrapper may be provided over the second heat-conducting element to provide the external surface of the smoking article. The additional wrapper may extend along all or just a part of the smoking article. As the second heat-conducting element is radially separated from the first heat-conducting element by the wrapper, the conductive transfer of heat from the first heat-conducting element to the second heat-conducting element is inhibited. As a result, the second heat-conducting element retains a lower temperature than the first heat-conducting element. The radiative losses of heat from the outer surfaces of the smoking article are reduced compared to a smoking article which does not have a second heat-conducting element around at least a portion of the first heat-conducting element.

The second heat-conducting element advantageously reduces the heat losses from the first heat-conducting element. The second heat-conducting element is formed of a heat conductive material which will increase in temperature during smoking of the smoking article, as heat is generated by the heat source. The increased temperature of the second heat-conducting element reduces the temperature differential between the first heat-conducting element and the overlying material such that the loss of heat from the first heat-conducting element can be reduced.

By reducing the heat losses from the first heat-conducting element, the second heat-conducting element advantageously helps to better maintain the temperature of the first heat-conducting element within the desired temperature range. The second heat-conducting element advantageously helps to more effectively use the heat from the heat source to warm the aerosol-forming substrate to the desired temperature range. In a further advantage, the second heat-conducting element helps maintain the temperature of the aerosol-forming substrate at a higher level. The second heat-conducting element in turn improves the generation of aerosol from the aerosol-forming substrate. Advantageously, the second heat-conducting element increases the overall delivery of aerosol to the user. In particular, it can be seen that the nicotine delivery can be significantly improved through the addition of the second heat-conducting element.

In addition, the second heat-conducting element has been found to advantageously extend the smoking duration for the smoking article so that a greater number of puffs can be taken.

In some preferred embodiments, the second heat-conducting element conducts heat along the smoking article from the heat source in the same way as the first heat-conducting element. The second heat-conducting element may therefore, in such embodiments, also improve the efficiency of the heat conduction from the heat source to the aerosol-forming substrate and therefore the heating of the aerosol-forming substrate.

The improvement to the conductive heat transfer achieved through the inclusion of a second heat-conducting element is particularly beneficial for smoking articles in which there is minimal convective heat transfer.

The provision of the second heat-conducting element over the wrapper provides further benefits in relation to the appearance of the smoking articles according to the invention, and in particular, the appearance of the smoking article during and after smoking. In certain cases, some discolouration of the wrapper in the region of the heat source is observed when the wrapper is exposed to heat from the heat source. The wrapper may additionally be stained as a result of the migration of the aerosol former from the aerosol-forming substrate into the wrapper. In smoking articles according to the invention, the second heat-conducting element can be provided over at least a part of the heat source and the adjacent part of the aerosol-forming substrate so that discolouration or staining is covered and no longer visible. The initial appearance of the smoking article can therefore be retained during smoking.

Where the one or more heat-conducting elements comprises a first heat-conducting element around and in contact with the heat source and the aerosol-forming substrate, as described above, the first heat-conducting element is preferably combustion resistant and oxygen restricting. In particularly preferred embodiments, the first heat-conducting element forms a continuous sleeve that tightly circumscribes the rear portion of the heat source and the front portion of the aerosol-forming substrate.

Preferably, the first heat-conducting element provides a substantially airtight connection between the heat source and the aerosol-forming substrate. This advantageously prevents combustion gases from the heat source being readily drawn into the aerosol-forming substrate through its periphery. Such a connection also minimises or substantially avoids convective heat transfer from the heat source to the aerosol-forming substrate by hot air drawn along the periphery.

Preferably the thickness of the first heat-conducting element is between about 5 microns and about 50 microns, more preferably between about 10 microns and about 30 microns and most preferably about 20 microns.

Preferably, the rear portion of the heat source overlain by the first heat-conducting element is between about 2 mm and about 8 mm in length, more preferably between about 3 mm and about 5 mm in length.

Preferably, the front portion of the heat source not overlain by the first heat-conducting element is between about 4 mm and about 15 mm in length, more preferably between about 5 mm and about 8 mm in length.

In certain embodiments, the first heat-conducting element overlies the entire length of the aerosol-forming substrate. In such embodiments, the downstream end of the first heat-conducting element may be aligned with the downstream end of the aerosol-forming substrate. Alternatively, the first heat-conducting element may extend beyond the aerosol-forming substrate in the downstream direction.

In other embodiments, the first heat-conducting element overlies only a front portion of the aerosol-forming substrate. In such embodiments, the aerosol-forming substrate extends beyond the first heat-conducting element in the downstream direction.

In embodiments in which the first heat-conducting element overlies only a front portion of the aerosol-forming substrate, the aerosol-forming substrate may extend at least about 3 mm downstream beyond the first heat-conducting element. For example, the aerosol-forming substrate may extend between about 3 mm and about 10 mm beyond the first heat-conducting element in the downstream direction. Alternatively, the aerosol-forming substrate may extend less than 3 mm downstream beyond the first heat-conducting element.

Where the smoking article comprises first and second heat-conducting elements, the second heat-conducting element is provided over at least a part of the first heat-conducting element and may extend around all or a part of the circumference of the smoking article. Preferably, the second heat-conducting element is in the form of a continuous sleeve circumscribing the smoking article, over a portion of at least the first heat-conducting element.

Preferably the thickness of the second heat-conducting element is between about 5 microns and about 50 microns, more preferably between about 5 microns and about 30 microns and most preferably between about 5 microns to about 20 microns. The thickness of the second heat-conducting element may be substantially the same as the thickness of the first heat-conducting element, or the heat-conducting elements may have a different thickness to each other.

The position and coverage of the second heat-conducting element may be adjusted relative to the first heat-conducting element and the underlying heat source and aerosol-forming substrate in order to control heating of the smoking article during smoking. The second heat-conducting element may be positioned over at least a part of the aerosol-forming substrate. Alternatively or in addition, the second heat-conducting element may be positioned over at least a part of the heat source. More preferably, the second heat-conducting element is provided over both a part of the aerosol-forming substrate and a part of the heat source, in a similar way to the first heat-conducting element.

The extent of the second heat-conducting element in relation to the first heat-conducting element in the upstream and downstream directions may be adjusted depending on the desired performance of the smoking article.

The second heat-conducting element may cover substantially the same area of the smoking article as the first heat-conducting element so that the heat-conducting elements extend along the same length of the smoking article. In this case, the second heat-conducting element preferably directly overlies the first heat-conducting element and fully covers the first heat-conducting element.

Alternatively, the second heat-conducting element may extend beyond the first heat-conducting element in the upstream direction, the downstream direction, or both the upstream and the downstream direction. Alternatively or in addition, the first heat-conducting element may extend beyond the second heat-conducting element in at least one of the upstream and downstream direction.

Preferably, the second heat-conducting element does not extend beyond the first heat-conducting element in the upstream direction. The second heat-conducting element may extend to approximately the same position on the heat source as the first heat-conducting element, such that the first and second heat-conducting elements are substantially aligned over the heat source. Alternatively, the first heat-conducting element may extend beyond the second heat-conducting element in an upstream direction. This arrangement may reduce the temperature of the heat source.

Preferably, the second heat-conducting element extends to at least the same position as the first heat-conducting element in the downstream direction. The second heat-conducting element may extend to approximately the same position on the aerosol-forming substrate as the first heat-conducting element such that the first and second heat-conducting elements are substantially aligned over the aerosol-forming substrate. Alternatively, the second heat-conducting element may extend beyond the first heat-conducting element in the downstream direction so that the second heat-conducting element covers the aerosol-forming substrate over a larger proportion of its length that then the first heat-conducting element. For example, the second heat-conducting element may extend by at least 1 mm beyond the first heat-conducting element, or at least 2 mm beyond the first heat-conducting element. Preferably however, the aerosol-forming substrate extends at least 2 mm downstream beyond the second heat-conducting element so that a rear portion of the aerosol-forming substrate remains uncovered by both heat-conducting elements.

The extent of the second heat-conducting element relative to the first heat-conducting element on the aerosol-forming substrate has a significant impact on the smoking performance of the smoking article. The coverage of the second heat-conducting element over the aerosol-forming substrate can therefore be adjusted in order to adjust the aerosol delivery profile of the smoking article.

In particular, it has been found that when the second heat-conducting element extends beyond the first heat-conducting element in a downstream direction, a more consistent puff-by-puff delivery of aerosol is provided during smoking. In particular, the aerosol delivery during the middle puffs is found to be reduced, thereby reducing the smoking intensity during these puffs in order to bring it more into line with the intensity at the start and end of smoking. It has also been found that the smoking duration is further increased.

When the second heat-conducting element extends beyond the first heat-conducting element over the aerosol-forming substrate, a larger area of the aerosol-forming substrate is covered by the second heat-conducting element. The heat is thereby dispersed through a greater volume of the aerosol-forming substrate, such that there is less of a temperature differential between different parts of the aerosol-forming substrate. This results in a decrease in the temperature of the front portion of the aerosol-forming substrate and an increase in the temperature of the downstream portions of the aerosol-forming substrate. It is believed that this is responsible for the observed effect on the puff-by-puff delivery of aerosol.

It has further been observed that adjustment of the extension of the second heat-conducting element beyond the first heat-conducting element in the downstream direction changes the time to the first puff of the smoking article. That is, the time delay between when the heat source begins heating the aerosol-forming substrate and the user receives the aerosol through the smoking article. In particular, the time to first puff will be increased by increasing the extension of the second heat-conducting element beyond the first heat-conducting element in the downstream direction.

Where the one or more heat-conducting elements comprises a single heat-conducting element printed onto an inner surface of the wrapper, the single heat-conducting element may be arranged as described above in relation to the first heat-conducting element. Where the one or more heat-conducting elements comprises a single heat-conducting element printed onto an outer surface of the wrapper, the single heat-conducting element may be arranged as described above in relation to the second heat-conducting element.

Either one or both of the first and second heat-conducting elements may comprise a patch of thermally conductive printable medium printed onto the wrapper. The second heat-conducting element may be formed of the same material as the first heat-conducting element, or a different material. Where only one of the first and second heat-conducting elements comprises a patch of thermally conductive printable medium, the other of the first and second heat-conducting elements may comprise a patch of heat-conducting sheet material applied to the wrapper. Suitable heat-conducting sheet materials include, but are not limited to: metal foil wrappers such as, for example, aluminium foil wrappers, steel wrappers, iron foil wrappers and copper foil wrappers; and metal alloy foil wrappers.

In certain embodiments, both the first and second heat-conducting elements are printed, with one on either side of the wrapper. That is, the one or more heat-conducting elements comprises a first heat conducting element comprising a patch of thermally conductive printable medium printed on the inner surface of the wrapper and a second heat conducting element comprising a patch of thermally conductive printable medium printed onto the outer surface of the wrapper.

In preferred embodiments, the first heat-conducting element comprises a patch of heat-conducting sheet material applied to the inner surface of the wrapper and the second heat-conducting element comprises a patch of thermally conductive printable medium printed on the outer surface of the wrapper. Alternatively, the first heat-conducting element may comprise a patch of thermally conductive printable medium printed on the inner surface of the wrapper while the second heat-conducting element comprises a patch of heat-conducting sheet material applied to the outer surface of the wrapper.

Where the first or second heat-conducting elements comprises a patch of heat-conducting sheet material applied to the surface of the wrapper, the patch of heat-conducting sheet material may be applied in any suitable process. For example, the patch of heat-conducting sheet material may be applied as described in WO-A-2009/112257.

The one or more heat-conducting elements may be arranged as disclosed in WO-A-2009/022232, WO-A-2009/112257, or WO-A-2013/120849.

The wrapper may comprise any suitable material. The wrapper may be a paper-like material. The smoking article wrapper material may be cigarette paper. The smoking article wrapper material may be another suitable, stiffer material, for example tipping paper.

In smoking articles according to the invention, heat is generated through a combustible heat source. Preferably, the heat source is a combustible carbonaceous heat source.

As used herein, the term "carbonaceous" is used to describe a heat source comprising carbon. Preferably, carbonaceous combustible heat sources according to the invention have a carbon content of at least about 35 percent, more preferably of at least about 40 percent, most preferably of at least about 45 percent by dry weight of the combustible heat source.

In some embodiments, the heat source of smoking articles according to the invention is a combustible carbon-based heat source. As used herein, the term carbon-based heat source' is used to describe a heat source comprised primarily of carbon.

Combustible carbon-based heat sources for use in smoking articles according to the invention may have a carbon content of at least about 50 percent, preferably of at least about 60 percent, more preferably of at least about 70 percent, most preferably of at least about 80 percent by dry weight of the combustible carbon-based heat source.

Smoking articles according to the invention may comprise combustible heat sources formed from one or more suitable carbon-containing materials. For example, smoking articles according to the invention may comprise combustible heat sources as disclosed in WO-A-2012/164077.

Preferably, the combustible heat source has a length of between about 7 mm and about 17 mm, more preferably of between about 7 mm and about 15 mm, most preferably of between about 7 mm and about 13 mm.

Preferably, the combustible heat source has a diameter of between about 5 mm and about 9 mm, more preferably of between about 7 mm and about 8 mm.

Preferably, the combustible heat source is of substantially uniform diameter. However, the combustible heat source may alternatively be tapered so that the diameter of the rear portion of the combustible heat source is greater than the diameter of the front portion thereof. Particularly preferred are combustible heat sources that are substantially cylindrical. The combustible heat source may, for example, be a cylinder or tapered cylinder of substantially circular cross-section or a cylinder or tapered cylinder of substantially elliptical cross-section.

Smoking articles according to the invention will include one or more airflow pathways along which air can be drawn through the smoking article for inhalation by a user.

In certain embodiments of the invention, the heat source comprises at least one longitudinal airflow channel, which provides one or more airflow pathways through the heat source. The term "airflow channel" is used herein to describe a channel extending along the length of the heat source through which air may be drawn through the smoking article for inhalation by a user. Such heat sources including one or more longitudinal airflow channels are referred to herein as "non-blind" heat sources.

The diameter of the at least one longitudinal airflow channel may be between about 1.5 mm and about 3 mm, more preferably between about 2 mm and about 2.5 mm. The inner surface of the at least one longitudinal airflow channel may be partially or entirely coated, as described in more detail in WO-A-2009/022232.

In alternative embodiments of the invention, no longitudinal airflow channels are provided in the heat source so that air drawn through the smoking article does not pass through any airflow channels along the heat source. Such heat sources are referred to herein as "blind" heat sources. Smoking articles including blind heat sources define alternative airflow pathways through the smoking article.

In smoking articles according to the invention comprising blind heat sources, heat transfer from the heat source to the aerosol-forming substrate occurs primarily by conduction and heating of the aerosol-forming substrate by convection is minimised or reduced. It is therefore particularly important with blind heat sources to optimise the conductive heat transfer between the heat source and the aerosol-forming substrate.

Preferably, smoking articles according to the invention comprise aerosol-forming substrates comprising at least one aerosol-former and a material capable of emitting volatile compounds in response to heating.

The at least one aerosol former may be any suitable known compound or mixture of compounds that, in use, facilitates formation of a dense and stable aerosol. The aerosol former is preferably resistant to thermal degradation at the operating temperature of the smoking article. Suitable aerosol-formers are well known in the art and include, for example, polyhydric alcohols, esters of polyhydric alcohols, such as glycerol mono-, di- or triacetate, and aliphatic esters of mono-, di- or polycarboxylic acids, such as dimethyl dodecanedioate and dimethyl tetradecanedioate. Preferred aerosol formers for use in smoking articles according to the invention are polyhydric alcohols or mixtures thereof, such as triethylene glycol, 1,3-butanediol and, most preferred, glycerine.

Preferably, the material capable of emitting volatile compounds in response to heating is a charge of plant-based material, more preferably a charge of homogenised plant-based material. For example, the aerosol-forming substrate may comprise one or more materials derived from plants including, but not limited to: tobacco; tea, for example green tea; peppermint; laurel; eucalyptus; basil; sage; verbena; and tarragon. The plant based-material may comprise additives including, but not limited to, humectants, flavourants, binders and mixtures thereof. Preferably, the plant-based material consists essentially of tobacco material, most preferably homogenised tobacco material.

Preferably, the aerosol-forming substrate has a length of between about 5 mm and about 20 mm, more preferably of between about 8 mm and about 12 mm. Preferably, the front portion of the aerosol-forming substrate surrounded by a heat-conducting element is between about 2 mm and about 10 mm in length, more preferably between about 3 mm and about 8 mm in length, most preferably between about 4 mm and about 6 mm in length. Preferably, the rear portion of the aerosol-forming substrate not surrounded by a heat-conducting element is between about 3 mm and about 10 mm in length. In other words, the aerosol-forming substrate preferably extends between about 3 mm and about 10 mm downstream beyond the heat-conducting element. More preferably, the aerosol-forming substrate extends at least about 4 mm downstream beyond the heat-conducting element.

The heat source and aerosol-forming substrate of smoking articles according to the invention may substantially abut one another. Alternatively, the heat source and aerosol-forming substrate of smoking articles according to the invention may be longitudinally spaced apart from one another one another.

Preferably smoking articles according to the invention comprise an airflow directing element downstream of the aerosol-forming substrate. The airflow directing element defines an airflow pathway through the smoking article. At least one air inlet is preferably provided between a downstream end of the aerosol-forming substrate and a downstream end of the airflow directing element. The airflow directing element directs the air from the at least one inlet towards the mouth end of the smoking article.

The airflow directing element may comprise an open-ended, substantially air impermeable hollow body. In such embodiments, the air drawn in through the at least one air inlet is first drawn upstream along the exterior portion of the open-ended, substantially air impermeable hollow body and then downstream through the interior of the open-ended, substantially air impermeable hollow body.

The substantially air impermeable hollow body may be formed from one or more suitable air impermeable materials that are substantially thermally stable at the temperature of the aerosol generated by the transfer of heat from the heat source to the aerosol-forming substrate. Suitable materials are known in the art and include, but are not limited to, cardboard, plastic, ceramic and combinations thereof.

In one preferred embodiment, the open-ended, substantially air impermeable hollow body is a cylinder, preferably a right circular cylinder.

In another preferred embodiment, the open-ended, substantially air impermeable hollow body is a truncated cone, preferably a truncated right circular cone.

The open-ended, substantially air impermeable hollow body may have a length of between about 7 mm and about 50 mm, for example a length of between about 10 mm and about 45 mm or between about 15 mm and about 30 mm. The airflow directing element may have other lengths depending upon the desired overall length of the smoking article, and the presence and length of other components within the smoking article.

Where the open-ended, substantially air impermeable hollow body is a cylinder, the cylinder may have a diameter of between about 2 mm and about 5 mm, for example a diameter of between about 2.5 mm and about 4.5 mm. The cylinder may have other diameters depending on the desired overall diameter of the smoking article.

Where the open-ended, substantially air impermeable hollow body is a truncated cone, the upstream end of the truncated cone may have a diameter of between about 2 mm and about 5 mm, for example a diameter of between about 2.5 mm and about 4.5 mm. The upstream end of the truncated cone may have other diameters depending on the desired overall diameter of the smoking article.

Where the open-ended, substantially air impermeable hollow body is a truncated cone, the downstream end of the truncated cone may have a diameter of between about 5 mm and about 9 mm, for example of between about 7 mm and about 8 mm. The downstream end of the truncated cone may have other diameters depending on the desired overall diameter of the smoking article. Preferably, the downstream end of the truncated cone is of substantially the same diameter as the aerosol-forming substrate.

The open-ended, substantially air impermeable hollow body may abut the aerosol-forming substrate. Alternatively, the open-ended, substantially air impermeable hollow body may extend into the aerosol-forming substrate. For example, in certain embodiments the open-ended, substantially air impermeable hollow body may extend a distance of up to 0.5L into the aerosol-forming substrate, where L is the length of the aerosol-forming substrate.

The upstream end of the substantially air impermeable hollow body is of reduced diameter compared to the aerosol-forming substrate.

In certain embodiments, the downstream end of the substantially air impermeable hollow body is of reduced diameter compared to the aerosol-forming substrate.

In other embodiments, the downstream end of the substantially air impermeable hollow body is of substantially the same diameter as the aerosol-forming substrate.

Where the downstream end of the substantially air impermeable hollow body is of reduced diameter compared to the aerosol-forming substrate, the substantially air impermeable hollow body may be circumscribed by a substantially air impermeable seal. In such embodiments, the substantially air impermeable seal is located downstream of the one or more air inlets. The substantially air impermeable seal may be of substantially the same diameter as the aerosol-forming substrate. For example, in some embodiments the downstream end of the substantially air impermeable hollow body may be circumscribed by a substantially impermeable plug or washer of substantially the same diameter as the aerosol-forming substrate.

The substantially air impermeable seal may be formed from one or more suitable air impermeable materials that are substantially thermally stable at the temperature of the aerosol generated by the transfer of heat from the combustible heat source to the aerosol-forming substrate. Suitable materials are known in the art and include, but are not limited to, cardboard, plastic, wax, silicone, ceramic and combinations thereof.

At least a portion of the length of the open-ended, substantially air impermeable hollow body may be circumscribed by an air permeable diffuser. The air permeable diffuser may be of substantially the same diameter as the aerosol-forming substrate. The air permeable diffuser may be formed from one or more suitable air permeable materials that are substantially thermally stable at the temperature of the aerosol generated by the transfer of heat from the combustible heat source to the aerosol-forming substrate. Suitable air permeable materials are known in the art and include, but are not limited to, porous materials such as, for example, cellulose acetate tow, cotton, open-cell ceramic and polymer foams, tobacco material and combinations thereof.

In one preferred embodiment, the airflow directing element comprises an open ended, substantially air impermeable, hollow tube of reduced diameter compared to the aerosol-forming substrate and an annular, substantially air impermeable seal of substantially the same outer diameter as the aerosol-forming substrate, which circumscribes a downstream end of the hollow tube.

The airflow directing element may further comprise an inner wrapper, which circumscribes the hollow tube and the annular substantially air impermeable seal.

The open upstream end of the hollow tube may abut a downstream end of the aerosol-forming substrate. Alternatively, the open upstream end of the hollow tube may be inserted or otherwise extend into the downstream end of the aerosol-forming substrate.

The airflow directing element may further comprise an annular air permeable diffuser of substantially the same outer diameter as the aerosol-forming substrate, which circumscribes at least a portion of the length of the hollow tube upstream of the annular substantially air impermeable seal. For example, the hollow tube may be at least partially embedded in a plug of cellulose acetate tow.

In another preferred embodiment, the airflow directing element comprises: an open ended, substantially air impermeable, truncated hollow cone having an upstream end of reduced diameter compared to the aerosol-forming substrate and a downstream end of substantially the same diameter as the aerosol-forming substrate.

The open upstream end of the truncated hollow cone may abut a downstream end of the aerosol-forming substrate. Alternatively, the open upstream end of the truncated hollow cone may be inserted or otherwise extend into the downstream end of the aerosol-forming substrate.

The airflow directing element may further comprise an annular air permeable diffuser of substantially the same outer diameter as the aerosol-forming substrate, which circumscribes at least a portion of the length of the truncated hollow cone. For example, the truncated hollow cone may be at least partially embedded in a plug of cellulose acetate tow.

Smoking articles according to the invention preferably further comprise an expansion chamber downstream of the aerosol-forming substrate and, where present, downstream of the airflow directing element. The inclusion of an expansion chamber advantageously allows further cooling of the aerosol generated by heat transfer from the heat source to the aerosol-forming substrate. The expansion chamber also advantageously allows the overall length of smoking articles according to the invention to be adjusted to a desired value, for example to a length similar to that of conventional cigarettes, through an appropriate choice of the length of the expansion chamber. Preferably, the expansion chamber is an elongate hollow tube.

Smoking articles according to the invention may also further comprise a mouthpiece downstream of the aerosol-forming substrate and, where present, downstream of the airflow directing element and expansion chamber. The mouthpiece may, for example, comprise a filter made of cellulose acetate, paper or other suitable known filtration materials. Preferably, the mouthpiece is of low filtration efficiency, more preferably of very low filtration efficiency. Alternatively or in addition, the mouthpiece may comprise one or more segments comprising absorbents, adsorbents, flavourants, and other aerosol modifiers and additives which are used in filters for conventional cigarettes, or combinations thereof.

According to a second aspect of the present invention, there is provided a method for applying heat conducting patches onto a web of smoking article wrapper material, each heat conducting patch being arranged to form a heat-conducting element of a smoking article when the web of smoking article wrapper material is subsequently used in the manufacture of smoking articles, the method comprising printing longitudinally spaced apart patches of a thermally conductive printable medium onto the web of smoking article wrapper material, wherein at least some of the patches comprise an area of thermally conductive printable medium extending continuously in the longitudinal direction for at least 2 mm.

With such a method, there is no risk of manufacturing downtime from misplaced heat conducting patches, as may be the case with existing processes, since the patches are printed directly onto the web of wrapper material. Further, the method is simpler and requires less machinery than known processes, such as in the method disclosed in WO-A-2009/112257, since no additional web of heat conducting material or its associated rollers are required. Furthermore, the appearance of smoking articles comprising patches of heat conducting material manufactured according to the present invention can be improved over smoking articles comprising patches of heat conducting material that are glued onto the wrapper, since the printed patches are less likely to form wrinkles in the wrapper.

As used herein, the term "width" refers to the dimension perpendicular to the longitudinal direction of the web of smoking article wrapper.

The web of smoking article wrapper material may comprise a ribbon, a strip or a band. In preferred embodiments, the web of smoking article wrapper material is a paper-like material. The smoking article wrapper material may be cigarette paper. The smoking article wrapper material may be another suitable, stiffer material, for example tipping paper.

As used herein, the term "smoking article" is used to mean not only conventional smoking articles in which the substrate, usually tobacco, is combusted, but also other smoking articles, for example heated smoking articles, in which the substrate is heated rather than combusted and which rely on aerosol formation from the heated substrate, and distillation-based smoking articles.

The step of printing may be performed using any suitable printing process. For example, the step of printing may be carried out using a movable printing head to print onto a moving or stationary web of smoking article wrapper material. For example, using a moveable printing nozzle operated using a CNC process. Preferably, the step of printing is carried out by conveying the web of smoking article wrapper material to a printing cylinder and printing the thermally conductive printable medium onto the web of smoking article wrapper material using the printing cylinder. Advantageously, the use of a printing cylinder allows the correct positioning of the patches on the web of smoking article wrapper material irrespective of the speed of operation, since the rotational speed of the printing cylinder is synchronised with the speed at which the web of smoking article wrapper is conveyed.

Preferably, the printing cylinder comprises a plurality of grooves for holding the thermally conductive printable medium, the grooves being arranged in a pattern corresponding to the desired pattern of heat conducting patches. The grooves may have any suitable size, shape or arrangement. The depth of the grooves may be adjusted according to the required thickness of printed heat conducting patch, the depth of the grooves taking into account a reduction in patch thickness as the printable medium dries. The required depth for a given thickness of patch will vary depending on the composition of the printable medium.

The heat conducting patches may be equally spaced on the web of smoking article wrapper material in the longitudinal direction. In such embodiments, the heat conducting patches are printed at an interval corresponding to the length of the wrapper of each smoking article manufactured using the web. For example, where the wrapper of each smoking article manufactured using the web has a length of about 50 mm, the heat conducting patches may be printed at intervals of about 50 mm.. Alternatively, the heat conducting patches may be spaced apart with different spacing between adjacent patches in the longitudinal direction. In certain embodiments, the heat conducting patches are spaced apart with two different, alternating spacings in the longitudinal direction. For example, the patches may be printed in pairs with the first patch in each pair being printed at an interval corresponding to twice the required length of the wrapper of a smoking article manufactured using the web, and with the two patches in each pair being separated by twice the required distance between the upstream end of the wrapper and the upstream end of the heat conducting element of a smoking article manufactured using the web.. This allows the web to be used to form double-length multi-segment components for smoking articles, the web being cut between adjacent heat-conducting patches in a subsequent process to form single-length multi-segment components for a smoking article. For example, where the wrapper of each smoking article manufactured using the web has a length of about 50 mm and the distance between the upstream end of the wrapper and the upstream end of the heat conducting element of the smoking article is 4 mm, the heat conducting patches may be printed with an interval of about 50 mm between the first patch in each pair and with a spacing of 8 mm between the two patches of each pair.

The longitudinal spacing between adjacent heat conducting patches is preferably at least 8 mm.

The step of printing may be carried out by any suitable apparatus. In certain embodiments, the step of printing is carried out using a conventional smoking article printing apparatus, such as described in EP1493339A1, EP1747730A1 or EP1512541A1, which has been modified to apply patches of a thermally conductive printable medium, for example by changing the shape and pattern of the grooves to correspond to the desired shape and pattern of the printed heat conducting patches and by changing the depth of the grooves to correspond to the required thickness of the printed heat conducting patches. In certain alternative embodiments, the step of printing is carried out using a conventional smoking article tipping apparatus which has been modified to apply patches of a thermally conductive printable medium, rather than strips of tipping glue.

The method may comprise applying first and second sets of longitudinally spaced apart patches of thermally conductive material onto the web of smoking article material, wherein at least one of the first and second sets is applied by printing patches of thermally conductive printable medium onto the web of smoking article wrapper material. In certain embodiments, one of the first and second sets of patches is applied by printing patches of thermally conductive printable medium, while the other of the first and second sets of patches is applied by attaching patches of a thermally conductive sheet material, for example using an adhesive. Suitable thermally conductive sheet materials include, but are not limited to: metal foil wrappers such as, for example, aluminium foil wrappers, steel wrappers, iron foil wrappers and copper foil wrappers; and metal alloy foil wrappers. The first and second sets of patches may be applied on the same side of the web of smoking article wrapper material. Alternatively, the first set of patches may be applied on a first side of the web of smoking article wrapper material and the second set of patches applied on a second, opposite side of the web of smoking article wrapper material.

In certain embodiments, the step of printing comprises printing first and second sets of longitudinally spaced apart patches of thermally conductive printable medium onto the web of smoking article wrapper material. In such embodiments, the step of printing may comprise printing a first set of longitudinally spaced apart patches of thermally conductive printable medium on a first side of the web of smoking article wrapper material and printing a second set of longitudinally spaced apart patches of thermally conductive printable medium on a second side of the web of smoking article wrapper material, the second side being opposite the first side. Thus, when the web of smoking article wrapper material is subsequently used to form a wrapper around a smoking article, the first and second sets of patches form heat conducting elements on either side of the wrapper to help retain and conduct heat within the smoking article.

The first and second sets of patches may be arranged in any suitable position relative to each other. In certain embodiments, the first and second sets of patches are longitudinally positioned such that at least part of each patch of one of the first or second sets overlies at least part of a patch of the other of the first or second set of patches in the longitudinal direction. That is, the first and second sets of patches are printed such that they are at least partially aligned in the longitudinally direction, albeit on opposite sides of the smoking article wrapper material. Preferably, the first and second sets of longitudinally spaced apart patches are substantially aligned. That is, the patches of the first and second sets are printed at the same position along the length, and on opposite sides, of the smoking article wrapper material. The patches of the second set may cover substantially the same area of the smoking article wrapper material as the patches of the first set so that both sets of patches extend along the same length of the smoking article wrapper material. In this case, the second set of patches preferably directly overlie and fully cover the first set of patches, albeit on opposite sides of the smoking article wrapper material.

Alternatively, the patches of the second set may extend beyond the patches of the first set in the upstream direction, the downstream direction, or both the upstream and the downstream direction. Alternatively or in addition, the patches of the first set may extend beyond the patches of the second set in at least one of the upstream and downstream direction.

The first set of patches and the second set of patches may be formed of the same material, or a different material.

In a third aspect of the present invention, there is provided a method for manufacturing a multi-segment component for a smoking article, the method comprising the steps of: applying heat conducting patches onto a web of wrapper material in accordance with any of the methods described above; feeding a stream of segments along a moving delivery path; compacting the stream of segments into groups of two or more different segments, each group corresponding to a discrete multi-segment component; wrapping the segments in the web of wrapper material such that one or more heat conducting patches are provided around at least one segment of each group; and cutting the web of wrapper material between adjacent groups to separate the discrete multi-segment components.

The method of the present invention may be utilised to manufacture smoking articles in a three stage process. The first stage is to form a multi-segment component, the second stage is to feed the multi-segment component and a second component or multi-segment component into a combining apparatus, and the third stage is to combine the multi-segment component with the second component or multi-segment component to form the smoking article.

Preferably, the segments on the delivery path have their longitudinal axes substantially aligned with each other and with the direction of movement of the delivery path. Such a linear forming process is advantageous since it causes minimal or no damage to the components within each first multi-segment component.

Preferably, the two or more different components comprise combustible carbonaceous heat sources and aerosol-forming substrates and wherein each group corresponding to a discrete multi-segment component comprises one of the combustible heat sources and one or more of the aerosol-forming substrates.

The step of feeding the stream of segments along the moving delivery path preferably comprises interleaving each of the different types of segments comprising the multi-segment component, such that the segments on the delivery path are in a desired and predetermined order. Preferably, the segments are interleaved along the moving delivery path such that the multi-segment component comprises a combustible heat source at a first end, an airflow directing segment at a second end and an aerosol-forming substrate between the combustible heat source and the airflow directing segment.

In an alternative embodiment, the multi-segment component further comprises an expansion chamber downstream of the airflow directing segment. In this alternative embodiment, the multi-segment component comprises four segments, preferably arranged such that the combustible heat source is provided at a first end, and the expansion chamber is provided at the second end. In this embodiment, the aerosol-forming substrate is provided adjacent the combustible heat source and the airflow directing segment is provided adjacent the expansion chamber.

The expansion chamber preferably forms a portion of the airflow pathway of the smoking article. The inclusion of an expansion chamber advantageously allows further cooling of the aerosol generated by heat transfer from the combustible heat source to the aerosol-forming substrate. The expansion chamber also advantageously allows the overall length of smoking articles according to the invention to be adjusted to a desired value, for example to a length similar to that of conventional cigarettes, through an appropriate choice of the length of the expansion chamber. In one embodiment, the expansion chamber may be a hollow tube having a cross-sectional shape equivalent to the cross-sectional shape.

In the alternative embodiment, the second multi-segment component preferably comprises a mouthpiece and a filter segment.

Preferably, in compacting the stream of segments into groups, there is a predefined space between a leading group of segments and a trailing group of segments.

In one embodiment, the step of compacting the stream of segments into groups of segments comprises: separating the stream of segments into groups, wherein each group corresponds to a discrete multi-segment component; compacting the segments within a group such that they abut one another; and setting the pre-defined space between a leading group of segments and a trailing group of segments.

Preferably, the step of compacting the segments within a group such that they abut one another comprises compacting the segments such that the aerosol-forming substrate is compressed by the combustible heat source and the airflow directing segment.

Preferably, the step of wrapping the segments in a web of smoking article wrapper material comprises wrapping the components in a paper web. Preferably, the web of smoking article wrapper material comprises a first set of heat-conducting patches on a first side of the web and in the segments are wrapped such that the first side of the web faces inwards and one or more of the first set of heat conducting patches is in contact with at least one segment of each group. Where each group comprises a combustible heat source and an aerosol-forming substrate, preferably, the segments are wrapped such that a heat conducting patch overlays at least a portion of the combustible heat source and at least a portion of the aerosol-forming substrate.

Preferably, the segments are substantially cylindrical, with a circular or elliptical cross section.

In any of the embodiments described above, the one or more heat conducting elements are preferably formed from material that has a bulk thermal conductivity of between 10 Watts per metre Kelvin (W/(m•K)) and about 500 Watts per metre Kelvin (W/(m•K)), more preferably between about 15 W/(m•K) and about 400 W/(m•K), at 23ºC and a relative humidity of 50% as measured using the modified transient plane source (MTPS) method. In the case of a heat conducting element formed from a patch of thermally conductive printable medium, the bulk thermal conductivity value is that of the patch when the printable medium has dried or cured.

As used herein, means plus function features may be expressed alternatively in terms of their corresponding structure.

Any feature relating to one aspect may be applied to other aspects, in any appropriate combination. In particular, method aspects may be applied to apparatus aspects, and vice versa. Furthermore, any, some or all features in one aspect can be applied to any, some or all features in any other aspect, in any appropriate combination.

It should also be appreciated that particular combinations of the various features described and defined in any aspects of the invention can be implemented or supplied or used independently.

The invention will be further described, by way of example only, with reference to the accompanying drawings in which:
Figure 1 shows a schematic longitudinal cross-section of a smoking article according to a first example, the smoking article having a first heat-conducting element;
Figure 2 shows a schematic longitudinal cross-section of a smoking article according to a second example, the smoking article having first and second heat-conducting elements;
Figure 3 shows a schematic representation of a first apparatus 301 for applying heat conducting patches to a web of wrapper material according to the invention;
Figure 4 shows a schematic representation of a second apparatus 401 for applying heat conducting patches to a web of wrapper material according to the invention;
Figure 5 shows a schematic representation of a paper web with a first example of heat-conducting patches applied according to the invention; and
Figure 6 shows a schematic representation of a paper web with a second example of heat-conducting patches applied according to the invention.

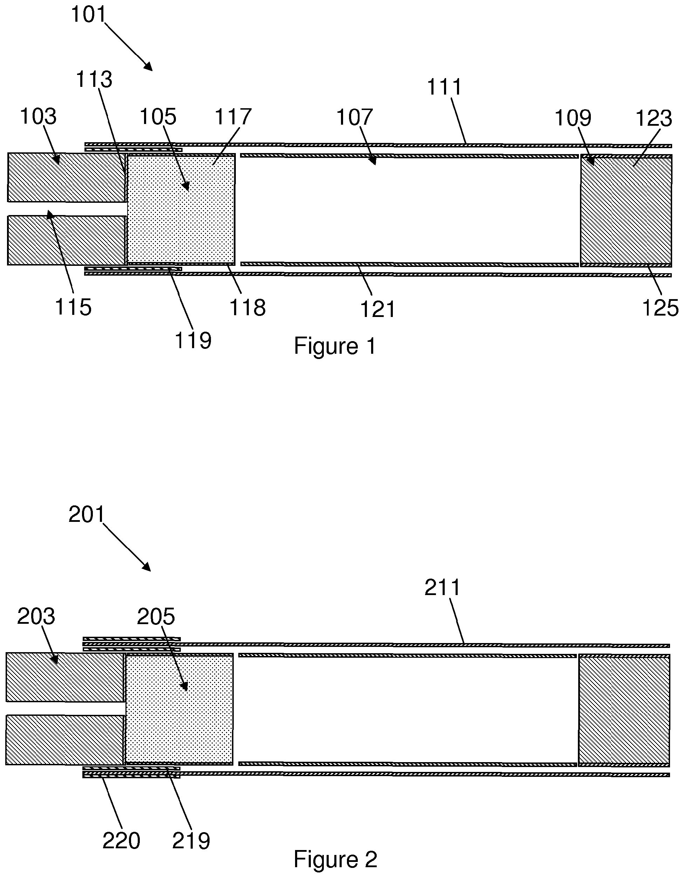

Figure 1 shows a first example of a smoking article comprising a heat-conducting element manufactured according to the present invention. The smoking article 101 comprises a combustible carbonaceous heat source 103, an aerosol-forming substrate 105, an elongate expansion chamber 107 and a mouthpiece 109 in abutting coaxial alignment. The combustible heat source 103, aerosol-forming substrate 105, elongate expansion chamber 107 and mouthpiece 109 are overwrapped in an outer paper wrapper 111 of cigarette paper of low air permeability. As shown in Figure 1, a non-combustible, gas-resistant, first barrier coating 113 is provided on substantially the entire rear face of the combustible carbonaceous heat source 103. In an alternative embodiment, a non-combustible, substantially air impermeable first barrier is provided in the form of a disc that abuts the rear face of the combustible carbonaceous heat source 103 and the front face of the aerosol-forming substrate 105. The combustible carbonaceous heat source 103 comprises a central airflow channel 115 that extends longitudinally through the combustible carbonaceous heat source 103 and the non-combustible, gas-resistant, first barrier coating 113. A gas-resistant, heat resistant, second barrier coating (not shown) is provided on the inner surface of the central airflow channel 115.

The aerosol-forming substrate 105 is located immediately downstream of the combustible heat source 103 and comprises a cylindrical plug of homogenised tobacco material 117 comprising glycerine as aerosol former and circumscribed by filter plug wrap 118. A heat-conducting element 119 surrounds and is in contact with a rear portion of the combustible heat source 103 and an abutting front portion of the aerosol-forming substrate 105. The heat-conducting element 119 conducts heat from the heat source 103 to the aerosol-forming substrate 105. As shown in Figure 1, a rear portion of the aerosol-forming substrate 105 is not surrounded by the heat-conducting element 119.

The elongate expansion chamber 107 is located downstream of the aerosol-forming substrate 105 and comprises a cylindrical open-ended tube of cardboard 121. The mouthpiece 109 is located downstream of the expansion chamber 107 and comprises a cylindrical plug of cellulose acetate tow 123 of very low filtration efficiency circumscribed by filter plug wrap 125. The mouthpiece 109 may be circumscribed by tipping paper (not shown).

In alternative embodiments (not shown), a heat insulating element is positioned around the wrapper 111 and the heat-conducting element 119 to retain heat within the smoking article.

Figure 2 shows a second example of a smoking article comprising a heat-conducting element manufactured according to the present invention. The smoking article 201 is substantially the same as the smoking article 101 described above in relation to Figure 1. However, rather than having a single heat-conducting element, smoking article 201 comprises a first heat-conducting element 219 that surrounds and is in contact with a rear portion of the combustible heat source 203 and an abutting front portion of the aerosol-forming substrate 205, and a second heat-conducting element 220 that surrounds and is in contact with the wrapper 211. The second heat-conducting element 220 is positioned over the first heat-conducting element 219 and is of the same length as the first heat-conducting element 219. The second heat-conducting element 220 therefore directly overlies the first heat-conducting element 219 with the wrapper 211 between them. The first heat-conducting element 219 conducts heat from the heat source 203 to the aerosol-forming substrate 205. The second heat-conducting element 220 retains heat within the smoking article 201 to help maintain the temperature of the first heat-conducting element 219 during smoking. This in turn helps maintain the temperature of the aerosol-forming substrate 205 to facilitate continued and enhanced aerosol delivery.

In alternative embodiments (not shown), a heat insulating element is positioned around the first and the second heat-conducting elements to retain heat within the smoking article.

The method of the present invention may be used during the manufacture of the smoking articles of Figures 1 and 2 to form the heat-conducting elements 119, 219 and 220 on the wrapper 111, 211 by printing heat conducting patches onto a paper web.

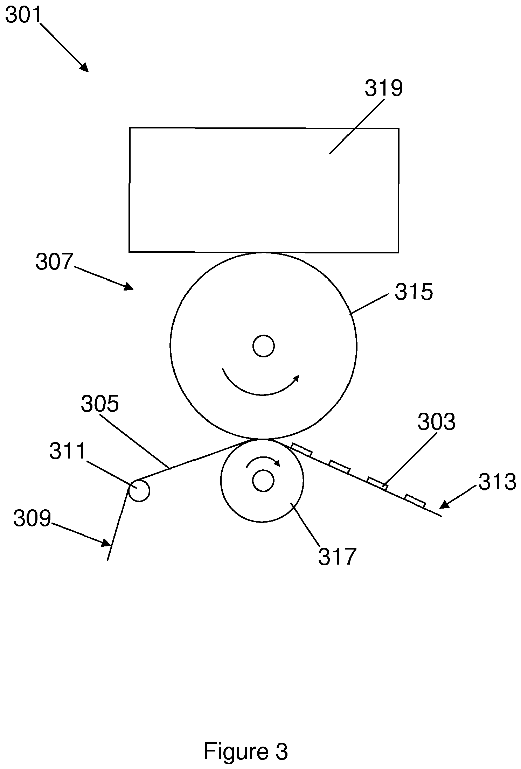

Figure 3 shows a schematic representation of a first apparatus 301 for the spaced application of heat conducting patches 303 to a paper web 305 according to the invention. The apparatus 301 comprises a printing unit 307, means for introducing the paper web in the form of paper input 309 and paper input drum 311, and paper output 313. The printing unit 307 comprises a printing cylinder 315, a counter roller 317, and a chamber 319. The chamber 319 has an inlet and an outlet (not shown) that connect the chamber 319 to a pressurised supply of heat-conductive printing ink and has an opening (not shown) that is sealed against the printing cylinder 315 by which the printing ink is transferred to the printing cylinder 315. The printing cylinder 315 comprises a series of grooves on its outer surface into which the ink is transferred.

Operation of the apparatus of Figure 3 is as follows. The paper web 305 is fed to paper input drum 311 from a paper feed bobbin (not shown) and on to the printing unit 307, where it is fed between the printing cylinder 315 and the counter roller 317. As the paper travels between the printing cylinder 315 and the counter roller 317, the heat conductive ink is printed from the cylinder 315 onto the paper web 305 to form a number of spaced apart heat conducting patches 303 on one side of the paper web 305. The spacing and pattern of the patches 303 correspond to the spacing and pattern of the grooves on the printing cylinder 315. The paper web 305 then leaves the apparatus 301 at paper output 313.

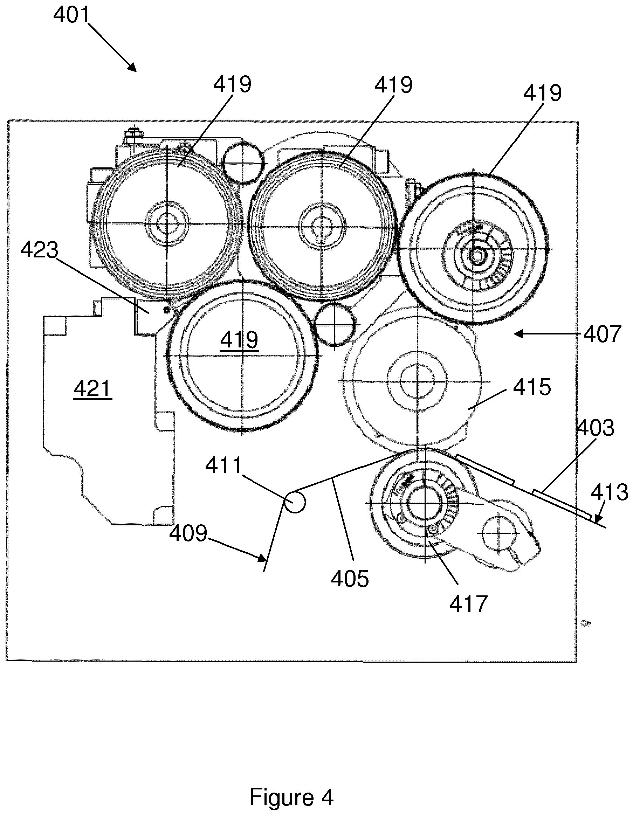

Figure 4 shows a schematic representation of a first apparatus 401 for the spaced application of heat conducting patches 403 to a paper web 405 according to the invention. The apparatus 401 comprises a printing unit 407, means for introducing the paper web in the form of paper input 409 and paper input drum 411, and paper output 413. The printing unit 407 comprises a printing cylinder 415, a counter roller 417, transfer rollers 419, pump 421, and nozzle 423. The pump 421 is connected to a supply of heat-conductive printing ink and to the nozzle 423 from which the ink is sprayed by the pump 421. The printing cylinder 415 comprises a series of grooves on its outer surface, the pattern of which corresponds to the desired pattern of patches 403 on the paper web 405. The diameter of the printing cylinder 415 may correspond to the interval between adjacent patches 403. For example, where the paper web 405 is used to make smoking articles having one printed patch 403, the diameter of the printing cylinder 415 may correspond to the length of the wrapper of each smoking article manufactured using the paper web 405.

Operation of the apparatus of Figure 4 is as follows. The pump 421 sprays a jet of heat conductive printing ink from the nozzle 423 between a first pair of transfer rollers 419. The ink is entrained between the first pair of transfer rollers 419 and is transferred via further transfer rollers 419, creating a homogenous layer of ink. The ink is transferred by the transfer rollers 419 to the printing cylinder 415. The paper web 405 is fed to paper input drum 411 from a paper feed bobbin (not shown) and on to the printing unit 407, where it is fed between the printing cylinder 415 and the counter roller 417. As the paper travels between the printing cylinder 415 and the counter roller 417, the heat conductive ink is transferred from the cylinder 415 onto the paper web 405 to form a number of spaced apart, printed heat conducting patches 403 on one side of the paper web 405. The arrangement of the patches 403 (that is, the spacing and pattern of the patches 403) is defined by the pattern of grooves on the surface of the printing cylinder 415. The number of grooves, their surface area and depth, as well as the pressure between the printing cylinder 415 and the counter roller 417 determines the quantity of ink applied to the paper web 405 and therefore the thickness of the resulting patches 403. The paper web 405 then leaves the apparatus 401 at paper output 413.

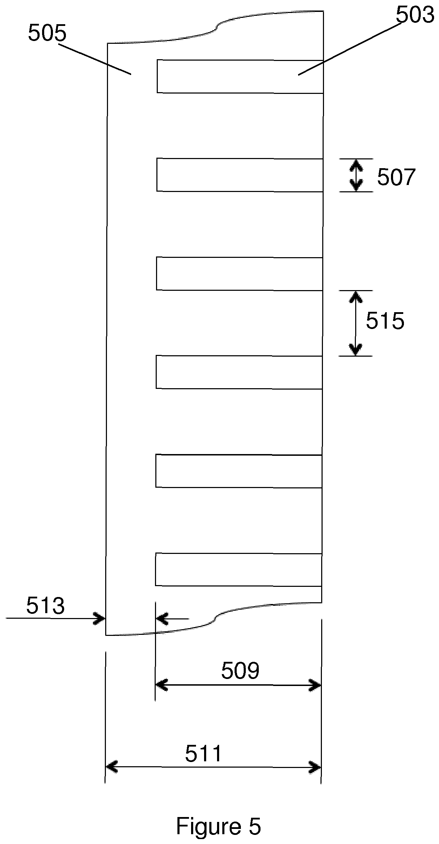

Figure 5 shows a schematic representation of a paper web 505 with a first example arrangement of heat-conducting patches 503 applied in accordance with the invention to form heat conducting elements 119 on smoking articles such as the smoking article shown in Figure 1. The patches 503 form heat conducting elements in the finished smoking articles. The length 507 of each patch 503 corresponds to the desired length of each heat conducting element. The width 509 of each patch 503 corresponds to the circumference of the finished smoking article. The width 511 of the web 505 corresponds to the circumference of the finished smoking article plus a margin 513 for gluing. In alternative embodiments (not shown), the width 509' of each patch 503' may be equal to the width 511' of the web 505', whereby the width 509' of each patch and the width 511' of the web 505' correspond to the circumference of the finished smoking article. The patches 503 are periodically printed on the web 505 with an even spacing 515. In smoking article manufacture, it is common to form a single length rod from one of more smoking article components wrapped in a paper web, before securing a filter mouthpiece at one end of the rod using tipping paper. The spacing 515 of the patches 503 corresponds to the spacing between heat conducting patches on adjacent rods.

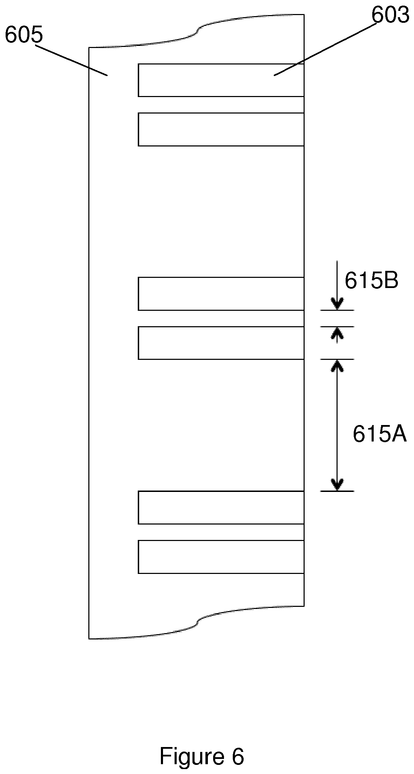

Figure 6 shows a schematic representation of a paper web 605 with a second example arrangement of heat-conducting patches 603 applied in accordance with the invention to form heat conducting elements 119 on smoking articles such as the smoking article shown in Figure 1. The paper web 605 is substantially the same as that shown in Figure 5. However, in Figure 6, the patches 603 are not equally spaced on the paper web 605. In fact, there are two different spacings 615A and 615B between the patches 603. This arrangement is particularly advantageous, as will now be described. It is common in smoking article manufacture to form double length rods of components for smoking articles wrapped in a paper web. Those double length rods are then cut in two, a double length filter mouthpiece is inserted in the centre and secured with tipping paper, and then the entire double length smoking article is cut to form two smoking articles. In this arrangement, each double length rod must have the segments in one half arranged in reverse order from the segments in the other half. This is so that, when the filter mouthpiece is inserted in the centre, two correct smoking articles are produced. Thus, the patches 603 on the paper web 605 will need to have two different spacings, as shown in Figure 6. The first spacing 615A corresponds to the spacing between patches in one double length rod. The second spacing 615B corresponds to the spacing between heat conducting patches on adjacent rods. The first and second spacings 615A and 615B are defined by the arrangement of grooves on the printing cylinder used to apply the patches 603.

Where spaced apart heat-conducting patches are required on both sides of the paper web, for example to provide a smoking article with first and second heat conducting elements as shown in Figure 2, the paper web is conveyed to a first apparatus for the application of a first set of heat conducting patches on a first side of the paper web before being conveyed to a second apparatus for the application of a second set of patches on a second, opposite, side of the paper web. The first and second apparatuses may both be arranged to print heat-conducting patches. The first and second apparatuses for applying heat conducting patches may be substantially identical. Alternatively, one of the apparatuses may be arranged to print the heat conducting patches (see, for example, Figures 3 and 4), while the other apparatus is arranged to cut and glue patches of heat conductive foil onto the web, for example as described in WO-A-2009/112257.

The embodiments shown in Figures 1 to 6 and described above illustrate but do not limit the invention. Other embodiments of the invention may be made without departing from the scope thereof, and it is to be understood that the specific embodiments described herein are not intended to be limiting.

## Claims

1. A smoking article (101, 201) comprising:
a carbonaceous combustible heat source (103);
an aerosol-forming substrate (105);
a wrapper (111) circumscribing at least the carbonaceous combustible heat source and the aerosol-forming substrate; and
one or more heat conducting elements (119) overlying a rear portion of the carbonaceous combustible heat source and an adjacent front portion of the aerosol-forming substrate,
wherein at least one heat conducting element comprises a patch (303) of thermally conductive printable medium printed onto the wrapper.

2. The smoking article (101, 201) of claim 1, wherein the one or more heat conducting elements (119) comprises a first heat conducting element (219) arranged on the inner surface of the wrapper (211) such that the first heat conducting element is in direct contact with the rear portion of the carbonaceous combustible heat source (203) and the adjacent front portion of the aerosol-forming substrate (205).

3. The smoking article (101, 201) of claim 2, wherein the one or more heat conducting elements (119) further comprises a second heat conducting element (220) around at least a portion of the first heat conducting element (219) and arranged on the outer surface of the wrapper (211).

4. The smoking article (101, 201) of any of claims 1 to 3, wherein at least one heat conducting element (119) comprises a patch (303) of thermally conductive printable medium printed onto the outer surface of the wrapper (111, 211.

5. A method for applying heat conducting patches (303) onto a web of smoking article wrapper material (305), each heat conducting patch being arranged to form a heat-conducting element (119) of a smoking article (101, 201) when the web of smoking article wrapper is subsequently used in the manufacture of smoking articles, the method comprising printing longitudinally spaced apart patches (303) of a thermally conductive printable medium onto the web of smoking article wrapper material (305), wherein at least some of the patches comprise an area of thermally conductive printable medium extending continuously in the longitudinal direction for at least 2 mm.

6. The method of claim 5, wherein the step of printing is carried out by conveying the web of smoking article wrapper material (305) to a printing cylinder (315) and transferring the thermally conductive printable medium onto the web of smoking article wrapper material using the printing cylinder.

7. The method of claim 6, wherein the printing cylinder (315) comprises a plurality of grooves for holding the thermally conductive printable medium, the grooves being arranged in a pattern corresponding to the desired pattern of patches (303).

8. The method of any of claims 5 to 7, wherein the step of printing comprises printing a first set of longitudinally spaced apart patches (303) of thermally conductive printable medium onto a first side of the web of smoking article wrapper material (305) and printing a second set of longitudinally spaced apart patches (303) of thermally conductive printable medium onto a second side of the web of smoking article wrapper material, the second side being opposite the first side.

9. The method of claim 8, wherein the first and second sets of longitudinally spaced apart patches (303) are substantially aligned.

10. The method of any of claims 5 to 9, wherein longitudinal spacing (515) between adjacent heat conducting patches (503) is at least 8 mm.

11. The method of any of claims 5 to 10, wherein each of the heat conducting patches (503) has a width (509) of at least about 10 mm, preferably of from about 15 mm to about 30 mm, more preferably from about 20 mm to about 25 mm.

12. A method for manufacturing a multi-segment component for a smoking article (101), the method comprising the steps of:
applying longitudinally spaced apart heat conducting patches (303) onto a web of smoking article wrapper material (305) in accordance with any of claims 5 to 11;
feeding a stream of segments along a moving delivery path;
compacting the stream of segments into groups of two or more different segments, each group corresponding to a discrete multi-segment component;
wrapping the segments in the web of smoking article wrapper material such that one or more heat conducting patches are provided around at least one segment of each group;
cutting the web of smoking article wrapper material between adjacent groups to separate the discrete multi-segment components.

13. The method of claim 12, wherein the two or more different segments in each group comprise a combustible carbonaceous heat source (103) and one or more aerosol-forming substrate (105) segments.

14. The method of claim 13, wherein the step of wrapping the segments in the web of smoking article wrapper material (305) is carried out such that one or more of the heat conducting patches (303) overlies at least part of the combustible carbonaceous heat source (103) and at least part of the one or more aerosol-forming substrates (105) of each group.

## Patentansprüche

1. Raucherartikel (101, 201), aufweisend:
eine kohlenstoffhaltige brennbare Wärmequelle (103);
ein aerosolbildendes Substrat (105);
eine Umhüllung (111), die mindestens die kohlenstoffhaltige brennbare Wärmequelle und das aerosolbildende Substrat abgrenzt; und
ein oder mehrere wärmeleitende Elemente (119), die über einem hinteren Teil der kohlenstoffhaltigen brennbaren Wärmequelle und einem angrenzenden Vorderteil des aerosolbildenden Substrats liegen,
wobei mindestens ein wärmeleitendes Element ein Patch (303) von auf die Umhüllung gedrucktem wärmeleitendem druckfähigem Medium aufweist.

2. Raucherartikel (101, 201) nach Anspruch 1, wobei das eine oder die mehreren wärmeleitenden Elemente (119) ein erstes wärmeleitendes Element (219) aufweist, das auf der Innenfläche der Umhüllung (211) derart angeordnet ist, dass das erste wärmeleitende Element in direktem Kontakt mit dem hinteren Teil der kohlenstoffhaltigen brennbaren Wärmequelle (203) und dem angrenzenden Vorderteil des aerosolbildenden Substrats (205) ist.

3. Raucherartikel (101, 201) nach Anspruch 2, wobei das eine oder die mehreren wärmeleitenden Elemente (119) weiter ein zweites wärmeleitendes Element (220) um mindestens einen Abschnitt des ersten wärmeleitenden Elements (219) herum und angeordnet auf der Außenfläche der Umhüllung (211) aufweist.

4. Raucherartikel (101, 201) nach einem der Ansprüche 1 bis 3, wobei mindestens ein wärmeleitendes Element (119) ein auf die Außenfläche der Umhüllung (111, 211) gedrucktes Patch (303) aus wärmeleitendem druckfähigem Medium aufweist.

5. Verfahren zum Aufbringen von wärmeleitenden Patches (303) auf eine Bahn aus Raucherartikelumhüllungsmaterial (305), wobei jedes wärmeleitende Patch ausgeführt ist, ein wärmeleitendes Element (119) eines Raucherartikels (101, 201) zu bilden, wenn die Bahn aus Raucherartikelumhüllung anschließend bei der Herstellung von Raucherartikeln verwendet wird, wobei das Verfahren das Drucken von in Längsrichtung voneinander beabstandeten Patches (303) eines wärmeleitenden druckfähigen Mediums auf die Bahn aus Raucherartikelumhüllungsmaterial (305) aufweist, wobei mindestens einige der Patches einen Bereich aus wärmeleitendem druckfähigem Medium aufweisen, das sich für mindestens 2 mm in der Längsrichtung kontinuierlich erstreckt.

6. Verfahren nach Anspruch 5, wobei der Schritt des Druckens durch Transportieren der Bahn aus Raucherartikelumhüllungsmaterial (305) zu einem Druckzylinder (315) und Übertragen des wärmeleitenden druckfähigen Mediums auf die Bahn aus Raucherartikelumhüllungsmaterial unter Verwendung des Druckzylinders ausgeführt wird.

7. Verfahren nach Anspruch 6, wobei der Druckzylinder (315) mehrere Nuten zum Halten des wärmeleitenden druckfähigen Mediums aufweist und die Nuten in einem Muster angeordnet sind, das dem gewünschten Muster von Patches (303) entspricht.

8. Verfahren nach einem der Ansprüche 5 bis 7, wobei der Schritt des Druckens das Drucken eines ersten Satzes von in Längsrichtung voneinander beabstandeten Patches (303) aus wärmeleitendem druckfähigem Medium auf eine erste Seite der Bahn aus Raucherartikelumhüllungsmaterial (305) und das Drucken eines zweiten Satzes von in Längsrichtung voneinander beabstandeten Patches (303) aus wärmeleitendem druckfähigem Medium auf eine zweite Seite der Bahn aus Raucherartikelumhüllungsmaterial aufweist und die zweite Seite der ersten Seite gegenüberliegt.

9. Verfahren nach Anspruch 8, wobei die ersten und zweiten Sätze von in Längsrichtung voneinander beabstandeten Patches (303) im Wesentlichen ausgerichtet sind.

10. Verfahren nach einem der Ansprüche 5 bis 9, wobei ein Längsabstand (515) zwischen angrenzenden wärmeleitenden Patches (503) mindestens 8 mm beträgt.

11. Verfahren nach einem der Ansprüche 5 bis 10, wobei jedes der wärmeleitenden Patches (503) eine Breite (509) von mindestens ungefähr 10 mm, bevorzugt von ungefähr 15 mm bis zu ungefähr 30 mm, mehr bevorzugt von ungefähr 20 mm bis zu ungefähr 25 mm, aufweist.

12. Verfahren zum Herstellen einer Mehrsegmentkomponente für einen Raucherartikel (101), wobei das Verfahren die Schritte aufweist:
Aufbringen von in Längsrichtung voneinander beabstandeten wärmeleitenden Patches (303) auf eine Bahn aus Raucherartikelumhüllungsmaterial (305) nach einem der Ansprüche 5 bis 11;
Zuführen eines Stroms von Segmenten entlang einem sich bewegenden Zufuhrweg;
Verdichten des Stroms von Segmenten in Gruppen von zwei oder mehr verschiedenen Segmenten, wobei jede Gruppe einer diskreten Mehrsegmentkomponente entspricht;
Hüllen der Segmente in die Bahn aus Raucherartikelumhüllungsmaterial, sodass ein oder mehrere wärmeleitende Patches um mindestens ein Segment jeder Gruppe herum vorgesehen sind;
Schneiden der Bahn aus Raucherartikelumhüllungsmaterial zwischen angrenzenden Gruppen, um die diskreten Mehrsegmentkomponenten zu trennen.

13. Verfahren nach Anspruch 12, wobei die zwei oder mehr verschiedenen Segmente in jeder Gruppe eine brennbare kohlenstoffhaltige Wärmequelle (103) und ein oder mehrere Segmente von aerosolbildendem Substrat (105) aufweisen.

14. Verfahren nach Anspruch 13, wobei der Schritt des Hüllens der Segmente in die Bahn aus Raucherartikelumhüllungsmaterial (305) derart ausgeführt wird, dass ein oder mehrere der wärmeleitenden Patches (303) über mindestens einem Teil der brennbaren kohlenstoffhaltigen Wärmequelle (103) und mindestens einem Teil von dem einen oder den mehreren aerosolbildende Substraten (105) jeder Gruppe liegen.

## Revendications

1. Article à fumer (101, 201) comprenant :
une source de chaleur combustible carbonée (103) ;
un substrat formant aérosol (105) ;
une enveloppe (111) entourant au moins la source de chaleur combustible carbonée et le substrat formant aérosol ; et
un ou plusieurs éléments thermoconducteurs (119) recouvrant une partie arrière de la source de chaleur combustible carbonée et une partie avant adjacente du substrat formant aérosol,
dans lequel au moins un élément thermoconducteur comprend une pièce (303) de support imprimable thermoconducteur imprimé sur l'enveloppe.

2. Article à fumer (101, 201) selon la revendication 1, dans lequel l'un ou plusieurs éléments thermoconducteurs (119) comprennent un premier élément thermoconducteur (219) agencé sur la surface intérieure de l'enveloppe (211) de sorte que le premier élément thermoconducteur soit en contact direct avec la partie arrière de la source de chaleur combustible carbonée (203) et la partie avant adjacente du substrat formant aérosol (205) .

3. Article à fumer (101, 201) selon la revendication 2, dans lequel l'un ou plusieurs éléments thermoconducteurs (119) comprennent en outre un deuxième élément thermoconducteur (220) autour d'au moins une partie du premier élément thermoconducteur (219) et agencé sur la surface extérieure de l'enveloppe (211).

4. Article à fumer (101, 201) selon l'une quelconque des revendications 1 à 3, dans lequel au moins un élément thermoconducteur (119) comprend une pièce (303) de support imprimable thermoconducteur imprimé sur la surface extérieure de l'enveloppe (111, 211).

5. Procédé d'application des pièces thermoconductrices (303) sur une bande de matière d'enveloppe de l'article à fumer (305), chaque pièce thermoconductrice est agencée pour former un élément thermoconducteur (119) d'un article à fumer (101, 201) lorsque la bande de l'enveloppe de l'article à fumer est ensuite utilisée dans la fabrication d'articles à fumer, le procédé comprenant l'impression de pièces espacées longitudinalement (303) d'un support imprimable thermoconducteur sur la bande de la matière d'enveloppe de l'article à fumer (305), où au moins certaines des pièces comprennent une zone de support imprimable thermoconducteur s'étendant continuellement dans la direction longitudinale sur au moins 2 mm.

6. Procédé selon la revendication 5, dans lequel l'étape d'impression est réalisée en transportant la bande de matière d'enveloppe de l'article à fumer (305) vers un cylindre d'impression (315) et en transférant le support imprimable thermoconducteur sur la bande de matière d'enveloppe de l'article à fumer à l'aide du cylindre d'impression.

7. Procédé selon la revendication 6, dans lequel le cylindre d'impression (315) comprend une pluralité de rainures pour maintenir le support imprimable thermoconducteur, les rainures étant agencées dans un motif correspondant au motif souhaité des pièces (303).

8. Procédé selon l'une quelconque des revendications 5 à 7, dans lequel l'étape d'impression comprend l'impression d'un premier ensemble de pièces espacées longitudinalement (303) du support imprimable thermoconducteur sur un premier côté de la bande de matière d'enveloppe de l'article à fumer (305) et d'impression d'un deuxième ensemble de pièces espacées longitudinalement (303) du support imprimable thermoconducteur sur un deuxième côté de la bande de matière d'enveloppe de l'article à fumer, le deuxième côté étant opposé au premier côté.

9. Procédé selon la revendication 8, dans lequel les premier et deuxième ensembles de pièces espacées longitudinalement (303) sont sensiblement alignées.

10. Procédé selon l'une quelconque des revendications 5 à 9, dans lequel l'espacement longitudinal (515) entre les pièces thermoconductrices adjacentes (503) est d'au moins 8 mm.

11. Procédé selon l'une quelconque des revendications 5 à 10, dans lequel chacun des pièces thermoconductrices (503) a une largeur (509) d'au moins environ 10 mm, de préférence d'environ 15 mm à environ 30 mm, plus préférablement d'environ 20 mm à environ 25 mm.

12. Procédé de fabrication d'un composant multi-segments pour un article à fumer (101), le procédé comprenant les étapes suivantes :
l'application de pièces thermoconductrices espacées longitudinalement (303) sur une bande de matière d'enveloppe de l'article à fumer (305) selon l'une quelconque des revendications 5 à 11 ;
l'alimentation en un flux de segments le long d'un trajet d'alimentation mobile ;
le compactage du flux de segments en groupes de deux ou plus segments différents, chaque groupe correspondant à un composant multisegment discret ;
l'enveloppement des segments dans la bande de la matière d'enveloppe de l'article à fumer de telle sorte qu'une ou plusieurs pièces thermoconductrices sont fournies autour d'au moins un segment de chaque groupe ;
la découpe de la matière d'enveloppe de l'article à fumer entre les groupes adjacents pour séparer les composants discrets multi-segments.

13. Procédé selon la revendication 12, dans lequel les deux ou plus segments différents dans chaque groupe comprennent une source de chaleur carbonée combustible (103) et un ou plusieurs segments de substrat formant aérosol (105).

14. Procédé selon la revendication 13, dans lequel l'étape d'enveloppement des segments dans la bande de la matière d'enveloppe de l'article à fumer (305) est effectuée de telle sorte qu'une ou plusieurs pièces thermoconductrices (303) recouvrent au moins une partie de la source de chaleur combustible carbonée (103) et au moins une partie de l'un ou plusieurs substrats formant aérosol (105) de chaque groupe.
